(19) Europäisches Patentamt

European Patent Office

Office européen des brevets

(11) EP 0 452 948 B1

(12) EUROPEAN PATENT SPECIFICATION

(45) Date of publication and mention
of the grant of the patent:
**24.07.1996 Bulletin 1996/30**

(51) Int Cl.⁶: **C12P 13/22**

(21) Application number: 91106275.0

(22) Date of filing: **19.04.1991**

(54) **Production process for L-phenylalanine**

Verfahren zur Herstellung von L-Phenylalanin

Procédé de préparation de L-phénylalanine

(84) Designated Contracting States:
**DE GB NL**

(30) Priority: **20.04.1990 JP 103003/90**

(43) Date of publication of application:
**23.10.1991 Bulletin 1991/43**

(73) Proprietor: **MITSUI TOATSU CHEMICALS, Inc.**
**Chiyoda-Ku Tokyo 100 (JP)**

(72) Inventors:
 • **Naito, Naokazu**
   **Ohmuta-shi, Fukuoka-ken (JP)**
 • **Koito, Mitsuo**
   **Yamato-gun, Fukuoka-ken (JP)**
 • **Ura, Daisuke**
   **Miike-gun, Fukuoka-ken (JP)**
 • **Fukuhara, Nobuhiro**
   **Ohmuta-shi, Fukuoka-ken (JP)**

(74) Representative: **Schüler, Horst, Dr.**
   **Patentanwalt,**
   **Kaiserstrasse 69**
   **D-60329 Frankfurt (DE)**

(56) References cited:
   **EP-A- 0 143 560**        **GB-A- 2 127 821**

**Description**

Background of the Invention

1) Field of the Invention

The present invention relates to a process for producing L-phenylalanine from cinnamic acid and an ammonia yielding substance by using L-phenylalanine ammonia lyase.

2) Description of the Related Art:

L-Phenylalanine is one of the essential amino acids for men, and has a very wide variety of utilities including its addition to drugs and feed. As a raw material for the production of Aspartame which is a sweetening agent of the dipeptide type, the demand for L-phenylalanine has been increasing in recent years. L-Phenylalanine is therefore useful in industry.

Production processes for L-phenylalanine, which have heretofore been known in the technical field of enzymatic production of L-phenylalanine, are disclosed in British Patent No. 1,489,468, Japanese Patent Publication No. 44474/1986, Japanese Patent Laid-Open Nos. 91890/1984, 133893/1985, 247395/1986 and 12297/1986, etc. These processes make use of the reverse reaction in the reversible reaction which takes place upon catalytic decomposition of L-phenylalanine to cinnamic acid and ammonia by L-phenylalanine ammonia lyase (hereinafter called "PAL").

In the process disclosed in British Patent No. 1,489,468, the enzymatic reaction is carried out in the presence of excess ammonia to increase the conversion from cinnamic acid to L-phenylalanine. This process made it possible for the first time to produce L-phenylalanine from cinnamic acid and ammonia by PAL.

A more advantageous reaction process is disclosed in Japanese Patent Publication No. 44474/1986, in which an ammonia concentration of at least about 3 mol/$\ell$ and a cinnamic acid concentration of 0.05-0.2 mol/$\ell$ are specifically used as reaction conditions.

These aforesaid patent publications however do not contain any disclosure about the stability of the enzyme in the reaction.

Conversely, it was reported by Hodgins et al. in Archives of Biochemistry and Biophysics, **149**, 91-96 (1972) that the enzymatic activity of PAL is inhibited by the binding of halogens to enzymatically-active sites of PAL. A similar teaching is found in Japanese Patent Laid-Open No. 91890/1984, in which use of a halogen-free ammonia yielding substance as an ammonia yielding substance is disclosed. This patent Laid-Open states that ammonium sulfate is preferred as a halogen-free ammonium yielding substance and that the reaction can be conducted preferably at an ammonia concentration of 1-5 mol/$\ell$.

Similarly, Japanese Patent Laid-Open No. 133893/1985 discloses that purification is facilitated by the use of ammonium carbonate as an ammonia yielding substance.

Japanese Patent Laid-Open No. 247395/1986 teaches that the conversion of cinnamic acid to L-phenylalanine can be increased by lowering the reaction temperature in a final stage of the reaction. Ammonium carbonate is used as the most preferable ammonium salt, and as reaction conditions, the concentration of cinnamic acid is about 0.01-1.0 mol/$\ell$, preferably 0.1-0.5 mol/$\ell$ while the concentration of ammonia is about 0.1-10.0 mol/$\ell$, preferably 1.0-8.0 mol/$\ell$.

Japanese Patent Laid-Open No. 12297/1986 discloses a process in which a reactant solution comprising at least 10 mol/$\ell$ of ammonia and 0.05-0.5 equivalent of carbonate ions relative to the amount of ammonia is used, the reaction being conducted at a cinnamic acid concentration not higher than 0.05 mol/$\ell$. This patent Laid-Open discloses that an advantage is available by the use of ammonium carbonate as an ammonia yielding substance, because, even at high ammonia concentrations, a stable activity of PAL can be maintained in the reactant solution containing ammonium carbonate and ammonium hydroxide.

Summary of the Invention

The present inventors proceeded with a further investigation of the reaction in which ammonium carbonate is used as an ammonium yielding substance. As a result, the following reaction-related problems were found:

1) A high ammonia concentration is needed to shift the equilibrium toward L-phenylalanine when L-phenylalanine is produced by the use of the enzyme, and in the reaction making use of ammonia carbonate as an ammonia yielding substance, the reaction can be carried out preferably at a pH range of 9.6-11.0, specially at pH 10.0 and at an ammonia concentration of at least 10 mol/$\ell$ in order to obtain a high reaction velocity. Under these preferred reaction conditions, PAL ordinarily undergoes salting out, and the resultant salted out PAL cannot contribute to the enzymatic reaction, thus lowering the effective concentration of PAL usable in the reaction mixture. (Similarly, using under reaction conditions in which ammonium sulfate, ammonium acetate or ammonium formate is used as an ammonia yielding substance, PAL also undergoes salting out so that the enzymatic reaction does not readily proceed.)

2) In contrast to the advantage derived from the use of ammonium carbonate as an ammonia yielding substance, i.e. a stable activity of PAL can be maintained even at a high ammonia concentration as described above, a disadvantage is that the reaction velocity is very low at ammonia concentrations not higher than 5 mol/$\ell$ where PAL does not undergo salting out even when ammonium carbonate is used as an ammonia yielding substance. This reaction velocity is extremely low even when compared with those available from the use of ammonium sulfate, ammonium acetate, ammonium formate and the like as ammonia yielding substances.

Thus, there is a serious drawback from the practical standpoint that, upon reaction in the reaction mixture comprising ammonium carbonate and ammonium hydroxide, the reaction velocity is low and that the salting out of PAL takes place.

The present inventors have carried out an extensive investigation with a view toward making improvements to the problems described above. As a result, it has been found that by using a reactant solution containing the ammonium salt of acetic acid, it is possible to significantly increase the reaction velocity compared to the reaction in a reactant solution containing ammonium carbonate and ammonium hydroxide. It is also possible to prevent salting out of PAL even at a pH of 10.0-10.6 and at an ammonia concentration of 10 mot/$\ell$ or higher.

The present invention therefore provides an industrially advantageous process for producing L-phenylalanine by using PAL, which process allows the reaction to be completed in a short period of time under advantageous conditions free from the salting out of PAL.

Detailed Description of the Preferred Embodiments

The present invention relates to a process for the production of phenylalanine, which comprises enzymatically producing L-phenylalanine from cinnamic acid and an ammonia yielding substance in the presence of PAL, said ammonia yielding substance comprising the ammonium salt of acetic acid, ammonium carbonate and ammonium hydroxide.

According to the process of the present invention, the reaction can be carried out without salting-out PAL even at high ammonia concentration. This is very advantageous for the reaction, thereby making it possible to conduct a better reaction than the conventionally-known reaction which is conducted in a reactant solution containing ammonium carbonate and ammonium hydroxide, but without any ammonium salt of a lower carboxylic acid.

PAL useful in the practice of the present invention is an enzyme which forms L-phenylalanine from cinnamic acid and an ammonia yielding substance.

PAL which exists in nature is known to be distributed not only in yeasts such as those belonging to the genus *Rhodosporidium* and microorganisms such as fungi but also in plants such as potato and parsley. These organisms can be used directly in the process of the present invention to obtain PAL activity. In addition, PAL-containing extracts from these organisms and their purified products of desired purity levels are also usable.

Further, a transformant having PAL activity may be also used in the present invention, which can be obtained by cloning a PAL structural gene from a PAL-producing organism in a vector; and introducing the PAL structural gene into a microorganism such as *Escherichia coli* or a cell line as a host so that PAL encoded in the PAL structural gene can be expressed in the resultant transformant. If necessary, the PAL structural gene is combined with materials necessary for the PAL expression such as promoter etc. in a vector and introduced into the host.

When a microorganism or cell line having intra-cell PAL-producing ability is employed, it is possible to use its culture (with cells contained therein), a dispersion of such cells, the cells obtained from the culture by centrifugal separation or the like, or a processed product of the cells (for example, washed cells, immobilized cells, disrupted cells, an autolysated cell product, a cell ultrasonicate or a cell lyophilyzate), or the like.

Where PAL is secreted, a supernatant culture or PAL recovered from the culture supernatant can also be used.

PAL employed in the present invention may also be those subjected to natural or artificial mutation provided that a desired degree of activity can be obtained.

The easiest preparation method of a reactant solution useful in the reaction of the present invention is to prepare at the same pH and ammonia concentration a solution containing ammonium carbonate and ammonium hydroxide and another solution containing the ammonium salt of acetic acid and ammonium hydroxide, and then to mix both the solutions with each other.

Described specifically, ammonium carbonate is dissolved in water or aqueous ammonia. The pH of the resultant solution is adjusted by adding aqueous ammonia or, if necessary, by sparging ammonia gas. By measuring the ammonia concentration of the resultant solution and then diluting the solution with water to a desired ammonia concentration, a solution (A) containing ammonium carbonate and ammonium hydroxide is prepared. Separately, the ammonium salt of acetic acid is dissolved in water or aqueous ammonia. The pH of the resultant solution is adjusted by adding aqueous ammonia or, if necessary, by sparging ammonia gas. By measuring the ammonia concentration of the resultant solution and then diluting the solution with water to a desired ammonia concentration, a solution (B) containing the ammonium salt of acetic acid and ammonium hydroxide is prepared. A reactant solution can be obtained by mixing the thus-prepared solutions (A) & (B) having the same pH and the same ammonia concentration at a preferred ratio. The pH and ammonia concentration of the solution obtained by the mixing are not different at all from the corresponding values before the mixing.

In other preparation methods, for example, wherein the ammonium salt of the acetic acid is added to a solution containing ammonium carbonate and ammonium hydroxide to adjust the pH, and wherein ammonium carbonate is added to a solution containing the ammonium salt of the acetic acid and ammonium hydroxide to adjust the pH, it is cumbersome and also difficult to prepare a reactant solution which contains ammonium carbonate, the ammonium salt of the acetic acid and ammonium hydroxide and which has the desired pH and ammonia concentration. These other preparation methods are therefore not preferred from a practical viewpoint. When the reactant solution is prepared by the above-described method in which two types of solutions (A) & (B) are mixed together, the reaction velocity increases provided that, as described in Example 1, the volumetric proportion (v/v) of the solution (B) containing the ammonium salt of the acetic acid and ammonium hydroxide is 5-90%, preferably 50-80% based on the whole reactant solution [(A) + (B)]. Proportions smaller than 5% result in only the same reaction velocity as that available in the reactant solution containing ammonium carbonate and ammonium hydroxide alone, whereas proportions greater than 90% lead to a significant reduction in the reaction velocity.

The reaction is carried out while controlling the concentration of ammonia in the reactant solution within 5-12 mol/$\ell$, preferably 10-12 mol/$\ell$. Use of a high ammonia concentration is advantageous for shifting the equilibrium toward L-phenylalanine.

The molar ratio of the acetic acid to carbonic acid in the reactant solution may be, for example, from 1:20 to 10:1, preferably from 1:1 to 5:1. Regarding the molar ratios of the acetic acid and carbonic acid to the concentration of ammonia in the reactant solution, it is preferable that X represented by the following formula falls within a range of from 1.0 to 7.0:

$$X = \frac{\text{Ammonia concentration (mol/}\ell\text{)}}{\text{Carbonic acid concentration (mol/}\ell\text{)} + \text{acetic acid concentration (mol/}\ell\text{)}}$$

When the reactant solution is prepared, for example, by the above-described method in which the two types of solutions (A) & (B) are mixed, the concentrations of the components in the respective solutions are adjusted in advance so that the concentrations of ammonia, carbonic acid and acetic acid in the reactant solution will equal their corresponding desired concentrations.

The reaction temperature is 10-40°C, with 25-35°C being preferred. The reaction velocity is slow at temperatures of 10°C or lower, while the enzymatic activity of PAL is reduced at temperature of 40°C and higher.

The reaction is carried out at a pH of 9.6-11.0, preferably 10.0-10.6.

Industrially-produced cinnamic acid can generally be used in the reaction.

Isolation of L-phenylalanine from the reaction mixture obtained in a reaction using cells having PAL activity can be carried out, for example, by a process which comprises the following steps:

(1) centrifuging the reaction mixture to remove the cells;
(2) heating the resultant solution to eliminate any excess ammonia;
(3) adding an acid to the solution to provide an acidic pH and centrifuging or filtering the thus pH-adjusted solution, whereby any remaining cinnamic acid precipitate is removed;
(4) causing the resultant solution to flow through an ion-exchange resin to adsorb L-phenylalanine, followed by eluting L-phenylalanine; and
(5) concentrating the resultant eluate containing L-phenylalanine, adjusting the pH of the residue to the isoelectric point (5.5) of L-phenylalanine, and then collecting precipitated L-phenylalanine by a separating process including filtration or the like.

The process of the present invention will hereinafter be described specifically by the following examples and comparative examples.

In the examples, the quantitative analyses of cinnamic acid and L-phenylalanine were conducted by a high performance liquid chromatographic method while using an ultraviolet absorption spectrophotometer provided as a detector. The detection was carried out at the wavelength of 223 nm. "FINEPAK SILC18"® (trade name; product of Japan Spectroscopic Co., Ltd.) was employed as a column. Measurement of the total concentration of ammonia in each reactant solution was conducted by the steam distillation-back titration method.

Example 1 (Initial reaction velocity in mixed reactant solution)

Prepared were Aqueous Solution A containing ammonium carbonate and ammonium hydroxide (carbonic acid concentration: 3.3 mol/$\ell$, ammonia concentration: 10 mol/$\ell$, pH 10), Aqueous Solution B containing ammonium acetate and ammonium hydroxide (acetic acid concentration: 6.3 mol/$\ell$, ammonia concentration: 10 mol/$\ell$, pH 10) and Aqueous Solution C containing ammonium formate and ammonium hydroxide (formic acid concentration: 6.5 mol/$\ell$, ammonia concentration: 10 mol/$\ell$, pH 10). Aqueous Solution A and Aqueous Solution B (or C) were mixed at the mixing ratios shown in Table 1, whereby Reactant Solution Nos. 1-8 were prepared.

Next, separately using the reactant solutions, production of PAL was conducted in the following manner.

Five milliliters of a suspension of PAL-producing cells (*Escherichia coli* strain MT 10410) in 100 mM Tris-HC$\ell$ buffer (pH 8.8), said suspension containing cells corresponding to 100 g of dry cells per $\ell$, were added to 50 g of each reactant solution. The resultant mixture was agitated under shaking at 30°C for 1 hour, followed by the addition of 0.37 g of cinnamic acid to the resultant solution to initiate the reaction. Upon an elapsed time of 90 minutes, the concentration of L-phenylalanine so produced was measured. The reaction velocity was determined from the amount of L-phenylalanine thus formed. Its ratio (i.e., the reaction velocity ratio) to the reaction velocity in Reactant Solution No. 1, which contained ammonium carbonate and ammonium hydroxide alone, is shown in Table 1, in which the latter velocity is taken as 100%.

Table 1

| Reactant solution No. | Mixing ratio (by volume) | | Reaction velocity ratio (%) |
|---|---|---|---|
| | A:B | A:C | |
| 1 | 100: 0 | - | 100 |
| 2 | 80:20 | - | 119 |
| 3 | 60:40 | - | 123 |
| 4 | 50:50 | - | 151 |
| 5 | 40:60 | - | 161 |
| 6 | 30:70 | - | 224 |
| 7 | 20:80 | - | 153 |
| 8 | - | 50:50 | 188 |

It is seen from Table 1 that the reaction velocities in Reactant Solution Nos. 2-7, each of which contained the combination of an ammonium salt of the acetic acid, ammonium carbonate and ammonium hydroxide were significantly higher compared to the reaction velocity in Reaction Solution No. 1 which contained ammonium carbonate and ammonium hydroxide. Reactant solution 8 serves as a Reference Example.

Example 2 (Salting-out in reactant solution)

PAL-producing cells (cells of *Escherichia coli* strain MT 10410) were diluted with 100 mM Tris-HC$\ell$ buffer (pH 8.8) to 50 g/$\ell$, whereby a suspension was prepared. The suspension was fed to a French press manufactured by SLM Instruments, Inc. and then disrupted (disruption condition: 800 bar). The liquid disruption mixture so formed was subjected to centrifugal separation for 30 minutes under 10,000 g. Two hundred milliliters of the resultant supernatant were added with ammonium sulfate to 28% saturation, followed by centrifugal separation. To the supernatant so formed, ammonium sulfate was added to 37% saturation followed by centrifugal separation again. To the precipitate, two hundred milliliters of Tris-HC$\ell$ buffer (100 mmol/$\ell$, pH 8.8) were added. The resultant solution was centrifuged to remove the insoluble matter. The thus-fractionated component (supernatant) was used as a crude enzyme solution (Sample I).

The preparation procedure for the crude enzyme solution was conducted at 10°C or below.

Twenty milliliter portion of the crude enzyme solution was filled in dialysis tube having a threshold molecular weight of 10,000. The dialysis tube was placed in Reactant Solution No. 4 (200 m$\ell$, free of cinnamic acid) prepared in Example 1 and then agitated under shaking at 20°C for 60 minutes. After it was treated for 1 hour, the enzyme solution was taken out of the tube and diluted with Reactant Solution No.4 to 20 m$\ell$. The diluted enzyme solution was subjected to centrifugal separation and the activity in the resultant supernatant (Sample II) was measured.

The measurement of the PAL activity was conducted in the following manner. To 0.5 m$\ell$ of tris-HC$\ell$ buffer (100 mmol/$\ell$, pH 8.8) were added 0.99 m$\ell$ of water and 0.5 m$\ell$ of an aqueous L-phenylalanine solution (100 mmol/$\ell$), and then a pre-incubation of the resultant solution was conducted at 30°C for 5 minutes. The enzyme solution (10 $\mu\ell$) was then added to initiate the reaction, and the reaction mixture was agitated under shaking at 30°C for 10 minutes. The concentration of cinnamic acid in the reaction mixture was measured by liquid chromatography. The activity was determined from the amount of cinnamic acid thus produced. The ratio of the thus-determined activity to the activity which had been obtained by using the same enzyme solution before the dialysis treatment, is shown in Table 2, in which the activity of Sample I is taken as 100%.

The same procedure as above was repeated except that Reactant Solution No.4 was replaced with Reactant Solution No.8.

Comparative Example 1 (Salting-out of PAL in centrifuged supernatant)

The procedures of Example 2 were repeated similarly except that the reactant solutions were changed to those shown in Table 2. The results are also shown in Table 2. In addition, the existence or non-existence of the PAL activity in each centrifuged precipitate after the dialysis was determined by suspending the precipitate in water and then measuring the activity in accordance with the activity measuring method described in Example 1 while using the suspension as an enzyme solution.

## Table 2

### (Activity in Centrifuged Supernatants at Various Ammonia Concentrations)

| | Reactant solution | pH | Ammonia concentration (mol/$\ell$) | Activity ratio* (%) |
|---|---|---|---|---|
| Ex. 2 | Reactant Solution No. 8 | 10.0 | 10.0 | 100.00 |
| Ex. 2 | Reactant Solution No. 4 | 10.0 | 10.0 | 100.00 |
| Comp. Ex. 1 | Ammonium carbonate-ammonium hydroxide reactant solution[a] | 10.0 | 10.0 | 75.00 |
| Comp. Ex. 1 | Ammonium sulfate-ammonium hydroxide reactant solution[b] | 10.0 | 10.0<br>7.5<br>5.0 | 0.00<br>10.10<br>95.50 |
| Comp. Ex. 1 | Ammonium acetate-ammonium hydroxide reactant solution[c] | 10.0 | 10.0<br>7.5<br>5.0 | 0.00<br>13.21<br>100.00 |
| Comp. Ex. 1 | Ammonium formate-ammonium hydroxide reactant solution[d] | 10.0 | 10.0<br>7.5<br>5.0 | 0.87<br>12.97<br>98.00 |

a) Carbonate ammonium concentration: 3.3 mol/$\ell$
b) Sulfate ammonium concentration: 3.5 mol/$\ell$
c) Acetate ammonium concentration: 6.3 mol/$\ell$
d) Formate ammonium concentration: 6.5 mol/$\ell$

$$*: \text{Activity ratio (\%)} = \frac{\text{Activity of Sample II}}{\text{Activity of Sample I}} \times 100$$

According to Table 2, no reduction in activity is observed in the supernatant in the case of the reactant solution (No.8) containing ammonium carbonate, ammonium formate and ammonium hydroxide or the reactant solution (No.

EP 0 452 948 B1

4) containing ammonium carbonate, ammonium acetate and ammonium hydroxide. This indicates that salting-out of PAL in a dialysis tube does not take place in such reactant solutions.

In each of the reactant solution containing ammonium sulfate and ammonium hydroxide, the reactant solution containing ammonium acetate and ammonium hydroxide and the reactant solution containing ammonium formate and ammonium hydroxide, the activity in the supernatant obtained by centrifuging the enzyme solution, which was contained in the dialysis tube after the treatment in the dialysis tube, decreased as the ammonia concentration increased. This means that salting-out of PAL took place in those reactant solutions when the concentrations of ammonia in the solutions were high. At an ammonia concentration of 5 mol/$\ell$, the activity in the centrifuged supernatant of the enzyme solution in the dialysis tube after the treatment in the dialysis tube was not different from the activity in the enzyme solution before the treatment in the dialysis tube. No salting-out of PAL therefore took place at ammonia concentrations of 5 mol/$\ell$ and lower in those reactant solutions. In addition, the salted-out precipitate was placed in a dialysis tube and dialyzed against the same tris-HC$\ell$ buffer as that employed for the above activity measurement to dissolve the precipitate. The PAL activity in the solution thus obtained in the dialysis tube was measured in a similar manner to that described above. Significant activity was observed. This indicates more clearly that PAL has been salted out.

Further, a reduction in activity was observed in the supernatant obtained from the solution in the dialysis tube after the dialysis treatment in the reactant solution containing ammonium carbonate and ammonium hydroxide. Significant activity was also observed in the solution which was obtained as in the above, namely, by re-dialyzing the precipitate against the same tris-HC$\ell$ buffer as that employed above for the measurement of the activity. This indicates that similar salting-out of PAL also takes place in the reactant solution containing ammonium carbonate and ammonium hydroxide. It is hence appreciated that this reaction condition is disadvantageous for the reaction.

Comparative Example 2 (Reaction velocities in various reactant solutions not using the combination of an ammonium salt of a lower carboxylic acid, ammonium carbonate and ammonium hydroxide.)

To 5-m$\ell$ portions of a suspension containing PAL-producing *Escherichia coli* MT 10410 cells corresponding to 100 g of dry cells per $\ell$, 50 g of the reactant solutions adjusted to the respective ammonia concentrations shown in Table 3 and Table 4 were added. Each mixture was agitated under shaking at 30°C for 1 hour to evenly suspend the cells. Thereafter, 0.37 g of cinnamic acid was added to initiate the reaction. After an elapsed time of 60 minutes, the concentration of L-phenylalanine formed in the reaction mixture was analyzed by liquid chromatography.

The results are shown in Table 3 and Table 4.

Table 3

| (Comparison of Reaction Velocities at Ammonia Concentration of 10 mol/$\ell$) | | | |
|---|---|---|---|
| | Ammonia concentration | pH | Concentration of L-phenylalanine accumulated* |
| Ammonium carbonate-ammonium hydroxide reactant solution[1] | 10 mol/$\ell$ | 10 | 0.705%(100)** |
| Ammonium sulfate-ammonium hydroxide reactant solution[2] | 10 mol/$\ell$ | 10 | 0.250% ( 35) |
| Ammonium acetate-ammonium hydroxide reactant solution[3] | 10 mol/$\ell$ | 10 | 0.000%( 0) |
| Ammonium formate-ammonium hydroxide reactant solution[4] | 10 mol/$\ell$ | 10 | 0.093%( 13) |

* Concentration of formed L-phenylalanine upon an elapsed time of 60 minutes.

** Each value in parentheses indicates the rate of the corresponding reaction velocity when the reaction velocity in the ammonium carbonate-ammonium hydroxide reactant solution is taken as 100.

7

Table 4

| (Comparison of Reaction Velocities at Ammonia Concentration of 5 mol/ℓ) | | | |
|---|---|---|---|
| | Ammonia concentration | pH | Concentration of L-phenylalanine accumulated* |
| Ammonium carbonate-ammonium hydroxide reactant solution[5]) | 5 mol/ℓ | 10 | 0.245%(100)** |
| Ammonium sulfate-ammonium hydroxide reactant solution[6]) | 5 mol/ℓ | 10 | 0.552% (225) |
| Ammonium acetate-ammonium hydroxide reactant solution[7]) | 5 mol/ℓ | 10 | 0.578%(235) |

In Tables 3 & 4:
* Concentration of formed L-phenylalanine upon an elapsed time of 60 minutes.

** Each value in parentheses indicates the rate of the corresponding reaction velocity when the reaction velocity in the ammonium carbonate-ammonium hydroxide reactant solution is taken as 100.
1) Carbonate ammonium concentration: 3.3 mol/ℓ
2) Sulfate ammonium concentration: 3.5 mol/ℓ
3) Acetate ammonium concentration: 6.3 mol/ℓ
4) Formate ammonium concentration: 6.5 mol/ℓ
5) Carbonate ammonium concentration: 2.9 mol/ℓ
6) Sulfate ammonium concentration: 1.5 mol/ℓ
7) Acetate ammonium concentration: 2.5 mol/ℓ

Table 3 indicates that the reaction does not proceed at the ammonia concentration of 10 mol/ℓ in the reactant solution containing ammonium formate and ammonium hydroxide, the reactant solution containing ammonium acetate and ammonium hydroxide and the reactant solution containing ammonium sulfate and ammonium hydroxide.

Table 4 indicates that the reaction velocity is extremely low in the reactant solution containing ammonium carbonate and ammonium hydroxide and having an ammonia concentration of 5 mol/ℓ compared with those in the reactant solution containing ammonium acetate and ammonium hydroxide and having an ammonia concentration of 5 mol/ℓ and in the reactant solution containing ammonium sulfate and ammonium hydroxide and having an ammonia concentration of 5 mol/ℓ.

*Escherichia coli* strain MT 10410 carrying a recombinant plasmid for PAL expression which was used in the examples is available from the deposit (Accession No. BP-1710) deposited on July 4, 1986, under the Budapest Treaty, with the Fermentation Research Institute of the Agency of Industrial Science and Technology, 1-3, Higashi 1-chome, Tsukuba-shi, Ibaraki-ken 305, Japan. This strain MT 10410 is described in European Patent Publication No. 0260919.

**Claims**

1. A process for the production of phenylalanine, which comprises reacting cinnamic acid and an ammonia yielding substance in the presence of L-phenylalanine ammonia lyase in a reactant solution to form L-phenylalanine, said ammonia yielding substance comprising the ammonium salt of acetic acid, ammonium carbonate and ammonium hydroxide.

2. The process of claim 1, wherein the concentration of ammonia in the reactant solution is 5-12 mol/ℓ.

3. The process of claim 1, wherein the molar ratio of the acetic acid to carbonic acid in the reactant solution ranges from 1:20 to 10:1.

4. The process of claim 1, wherein the concentrations of ammonia, the acetic acid and carbonic acid in the reactant solution are adjusted to control, within a range of 1.0-7.0, the value of X represented by the following formula:

$$X = \frac{\text{Ammonia concentration (mol/ℓ)}}{\text{Carbonic acid concentration (mol/ℓ) + acetic acid concentration (mol/ℓ)}}$$

5. The process of claim 1, wherein the pH of the reactant solution is in a range of 9.6-11.0.

6. The process of claim 1, wherein the concentration of ammonia in the reactant solution is 10-12 mol/ℓ.

**7.** the process of claim 1, wherein the molar ratio of the acetic acid to carbonic acid in the reactant solution ranges from 1:1 to 5:1.

**8.** The process of claim 1, wherein the pH of the reactant solution is in a range of 10.0-10.6.

**9.** The process of claim 4, wherein the molar ratio of the acetic acid to carbonic acid in the reactant solution ranges from 1:1 to 5:1.

**10.** The process of claim 4, wherein the pH of the reactant solution is in a range of 10.0-10.6

**11.** The process of claim 4, wherein the concentration of ammonia in the reactant solution is 10-12 mol/$\ell$.

**12.** The process of claim 9, wherein the pH of the reactant solution is in a range of 9.6-11.0.

**Patentansprüche**

**1.** Ein Verfahren zur Herstellung von Phenylalanin, das umfaßt, daß Zimtsäure und eine Ammoniak liefernde Substanz in Anwesenheit von L-Phenylalaninammoniaklyase in einer Reaktionslösung umgesetzt werden, um L-Phenylalanin zu bilden, wobei die Ammoniak liefernde Substanz das Ammoniumsalz von Essigsäure, Ammoniumcarbonat und Ammoniumhydroxid umfaßt.

**2.** Das Verfahren nach Anspruch 1, bei dem die Konzentration von Ammoniak in der Reaktionslösung 5 - 12 Mol/l beträgt.

**3.** Das Verfahren nach Anspruch 1, bei dem das molare Verhältnis der Essigsäure zu Kohlensäure in der Reaktionslösung von 1:20 bis 10:1 reicht.

**4.** Das Verfahren nach Anspruch 1, bei dem die Konzentrationen von Ammoniak, der Essigsäure und Carbonsäure in der Reaktionslösung eingestellt werden, um den Wert von X, der durch die folgende Formel ausgedrückt wird:

$$X = \frac{\text{Ammoniakkonzentration (Mol/l)}}{\text{Kohlensäurekonzentration (Mol/l) + Essigsäurekonzentration (Mol/l)}}$$

innerhalb eines Bereiches von 1,0 - 7,0 zu steuern.

**5.** Das Verfahren nach Anspruch 1, bei dem der pH der Reaktionslösung in einem Bereich von 9,6 - 11,0 liegt.

**6.** Das Verfahren nach Anspruch 1, bei dem die Konzentration von Ammoniak in der Reaktionslösung 10 - 12 Mol/l beträgt.

**7.** Das Verfahren nach Anspruch 1, bei dem das molare Verhältnis der Essigsäure zu Kohlensäure in der Reaktionslösung von 1:1 bis 5:1 reicht.

**8.** Das Verfahren nach Anspruch 1, bei dem der pH der Reaktionslösung in einem Bereich von 10,0 - 10,6 liegt.

**9.** Das Verfahren nach Anspruch 4, bei dem das molare Verhältnis der Essigsäure zu Kohlensäure in der Reaktionslösung von 1:1 bis 5:1 reicht.

**10.** Das Verfahren nach Anspruch 4, bei dem der pH der Reaktionslösung in einem Bereich von 10,0 - 10,6 liegt.

**11.** Das Verfahren nach Anspruch 4, bei dem die Konzentration von Ammoniak in der Reaktionslösung 10 - 12 Mol/l beträgt.

**12.** Das Verfahren nach Anspruch 9, bei dem der pH der Reaktionslösung in einem Bereich von 9,6 - 11,0 liegt.

**Revendications**

**1.** Procédé pour la production de phénylalanine, qui comprend la mise en réaction d'acide cinnamique et d'une substance cédant de l'ammoniac en présence de lyase ammoniacale L-phénylalanine dans une solution réactive pour former la L-phénylalanine, la substance cédant l'ammoniac comprenant le sel d'ammonium de l'acide acétique, le carbonate d'ammonium et l'hydroxyde d'ammonium.

2. Procédé selon la revendication 1, dans lequel la concentration d'ammoniac dans la solution réactive et de 5-12 moles/ℓ.

3. Procédé selon la revendication 1, dans lequel le rapport molaire entre l'acide acétique et l'acide carbonique dans la solution réactive varie de 1:20 à 10:1.

4. Procédé selon la revendication 1, dans lequel les concentrations d'ammoniac, l'acide acétique et l'acide carbonique dans la solution réactive sont ajustées pour réguler, à l'intérieur d'une plage de 1,0-7,0, la valeur de X représenté par la formule suivante :

$$X = \frac{\text{Concentration d'ammoniac (mole/}\ell\text{)}}{\text{Concentration acide carbonique (mole/}\ell\text{)} + \text{Concentration acide acétique (mole/}\ell\text{)}}$$

5. Procédé selon la revendication 1, dans lequel le pH de la solution réactive se situe dans une plage de 9,6-11,0.

6. Procédé selon la revendication 1, dans lequel la concentration d'ammoniac dans la solution réactive est de 10-12 mole/ℓ.

7. Procédé selon la revendication 1, dans lequel le rapport molaire entre l'acide acétique et l'acide carbonique dans la solution réactive se situe de 1:1 à 5:1.

8. Procédé selon la revendication 1, dans lequel le pH de la solution réactive se situe dans une plage de 10,0-10,6.

9. Procédé selon la revendication 4, dans lequel le rapport molaire entre l'acide acétique et l'acide carbonique dans la solution réactive varie de 1:1 à 5:1.

10. Procédé selon la revendication 4, dans lequel le pH de la solution réactive se situe dans une plage de 10,0-10,6.

11. Procédé selon la revendication 4, dans lequel la concentration d'ammoniac dans la solution réactive est de 10-12 mole/ℓ.

12. Procédé selon la revendication 9, dans lequel le pH de la solution réactive se situe dans une plage de 9,6-11,0.